(19) Europäisches Patentamt / European Patent Office / Office européen des brevets

(11) **EP 4 099 017 A1**

(12) **EUROPEAN PATENT APPLICATION**
published in accordance with Art. 153(4) EPC

(43) Date of publication:
**07.12.2022 Bulletin 2022/49**

(21) Application number: **20916624.8**

(22) Date of filing: **25.12.2020**

(51) International Patent Classification (IPC):
**G01N 33/531** *(2006.01)*        **G01N 33/543** *(2006.01)*

(52) Cooperative Patent Classification (CPC):
**G01N 33/531; G01N 33/543**

(86) International application number:
**PCT/JP2020/048659**

(87) International publication number:
**WO 2021/153127 (05.08.2021 Gazette 2021/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.01.2020 JP 2020014702**

(71) Applicant: **FUJIFILM Corporation
Tokyo 106-8620 (JP)**

(72) Inventors:
• **ABURAYA Yoshihiro
  Ashigarakami-gun, Kanagawa 258-8538 (JP)**
• **KATADA Junichi
  Ashigarakami-gun, Kanagawa 258-8538 (JP)**

(74) Representative: **Hughes, Andrea Michelle
Dehns Germany
Theresienstraße 6-8
80333 München (DE)**

(54) **IMMUNOLOGICAL TEST METHOD**

(57)     There is provided an immunological test method having high detection sensitivity. The immunological test method includes a concentration step of mixing a solution capable of containing an antigen, a superabsorbent polymer, and a labeled antibody against the antigen, to obtain a concentrated and mixed solution which is a concentrated mixed solution containing composite bodies of the antigen and the labeled antibody and a detection step of detecting the composite bodies in the concentrated and mixed solution using an antigen-antibody reaction, in which a swelling ratio of the superabsorbent polymer is more than 0.2 g/g and less than 800 g/g.

FIG. 1

**Description**

**BACKGROUND OF THE INVENTION**

1. Field of the Invention

The present invention relates to an immunological test method.

2. Description of the Related Art

Immunological test methods (particularly, immunochromatography) are frequently used these days since the operation is easy and measurement can be carried out in a short time. For example, in a case where an antigen such as influenza virus is detected by immunochromatography, the following operations are carried out. First, a label modified with an antibody (an antibody modified with a label) (a labeled antibody) is prepared and mixed with a specimen containing an antigen. The labeled antibody binds to an antigen, whereby composite bodies are formed. In this state, in a case where these composite bodies are spread on an insoluble carrier having a detection line (a test line) onto which an antibody that specifically reacts with an antigen is applied, the composite bodies react with the antibody on the detection line and are captured, and detection is confirmed visually or in other manners. Examples of such immunochromatography include the immunochromatography disclosed in JP5728453B.

**SUMMARY OF THE INVENTION**

These days, an immunodiagnostic method applicable to a sample solution having an extremely low antigen concentration is desired, and regarding the immunological test method such as immunochromatography, there is also a demand for a method having higher sensitivity than the method in the related art (for example, the method disclosed in JP5728453B).
In consideration of the above circumstances, an object of the present invention is to provide an immunological test method having high detection sensitivity.
As a result of diligent studies on the above-described problems, inventors of the present invention have found that the above-described problems can be solved by using a sample solution concentrated by a predetermined method and have reached the present invention. That is, the inventors of the present invention have found that the object can be achieved by the following configurations.

(1) An immunological test method comprising a concentration step of mixing a solution capable of containing an antigen, a superabsorbent polymer, and a labeled antibody against the antigen, to obtain a concentrated and mixed solution which is a concentrated mixed solution containing composite bodies of the antigen and the labeled antibody and a detection step of detecting the composite bodies in the concentrated and mixed solution using an antigen-antibody reaction, in which a swelling ratio of the superabsorbent polymer is more than 0.2 g/g and less than 800 g/g.
(2) The immunological test method according to (1), in which the solution capable of containing an antigen is urine.
(3) The immunological test method according to (2), in which a concentration of urea in the concentrated and mixed solution is 5 times or less with respect to a concentration of urea in the solution capable of containing an antigen.
(4) The immunological test method according to any one of (1) to (3), in which a water absorption rate of the superabsorbent polymer is 0.01 g/min or more and 40 g/min or less per 1 g of the superabsorbent polymer.
(5) The immunological test method according to any one of (1) to (4), in which the immunological test method is immunochromatography.
(6) The immunological test method according to any one of (1) to (5), in which the labeled antibody is an antibody-modified gold colloid particle which is a monoclonal antibody capable of binding to the antigen labeled with a gold colloid particle.
(7) The immunological test method according to any one of (1) to (6), in which the superabsorbent polymer is a polyacrylic acid-based, polyacrylamide-based, cellulose-based, or polyethylene oxide-based polymer.
(8) The immunological test method according to any one of (1) to (7), in which a particle diameter of the superabsorbent polymer is 5 mm or less.
(9) The immunological test method according to any one of (1) to (8), in which the concentration step is a step of mixing casein and tricine in addition to the solution capable of containing an antigen, the superabsorbent polymer, and the labeled antibody.

As described below, according to the present invention, it is possible to provide an immunological test method having high detection sensitivity.

**BRIEF DESCRIPTION OF THE DRAWINGS**

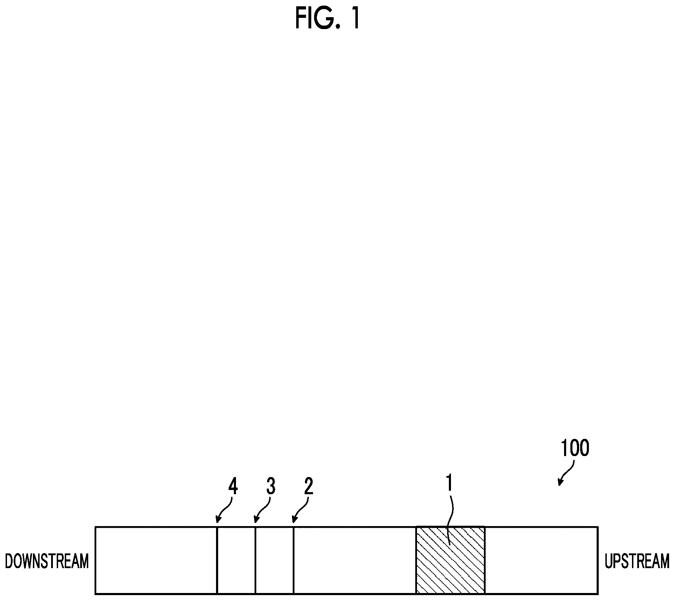

Fig. 1 is a schematic view illustrating an aspect of an insoluble carrier that is used in a method according to the embodiment of the present invention.

**DESCRIPTION OF THE PREFERRED EMBODIMENTS**

An immunological test method according to an embodiment of the present invention will be described below.
In the present specification, the numerical value range indicated by using "to" means a range including the numerical values before and after "to" as the lower limit value and the upper limit value, respectively. In addition, in the present specification, one kind of each component may be used alone, or two or more kinds thereof may be used in combination. In a case where two or more kinds of each component are used in combination, the content of the component indicates a total content unless otherwise specified. Further, in the present specification, "the detection sensitivity and the signal/noise ratio (the S/N ratio) are further improved" is also described as "the effects and the like of the present invention are more excellent".
The immunological test method according to the embodiment of the present invention (hereinafter, also referred to as "the method according to the embodiment of the present invention") is an immunological test method including a concentration step of mixing a solution capable of containing an antigen, a superabsorbent polymer, and a labeled antibody against the above antigen, to obtain a concentrated and mixed solution which is a concentrated mixed solution containing composite bodies of the above antigen and the labeled antibody and a detection step of detecting the composite bodies in the concentrated and mixed solution using an antigen-antibody reaction, in which a swelling ratio of the superabsorbent polymer is more than 0.2 g/g and less than 800 g/g.
It is presumed that since the method according to the embodiment of the present invention has such a configuration, the above effects can be obtained. The reason for this is not clear; however, it is conceived to be as follows.
As described above, in the method according to the embodiment of the present invention, a solution capable of containing an antigen (a sample solution), a superabsorbent polymer having a specific swelling ratio, and a labeled antibody against the above-described antigen are mixed. In a case where the sample solution and the superabsorbent polymer are mixed, the water in the sample solution is incorporated into the superabsorbent polymer, whereas an antigen or composite bodies of an antigen and a labeled antibody in the sample solution are hardly incorporated into the superabsorbent polymer since the antigen has a hydrodynamic radius of a certain degree and thus the network structure on the surface of the superabsorbent polymer exhibits a sieving effect. As a result, the concentration of the sample solution and the antigen-antibody reaction proceed at the same time, and the composite bodies are formed in a concentrated state, which leads to an improvement in the detection sensitivity.
On the other hand, a sample solution generally contains impurities such as low molecular weight components and salts. For example, in a case where the sample solution is urine, it contains impurities such as urea. From the studies by inventors of the present invention, it was found that in a case where these impurities are concentrated together with an antigen, the antigen-antibody reaction in the detection step is inhibited and the detection sensitivity is decreased. That is, it is known that the effect of improving the detection sensitivity by concentration cannot be sufficiently obtained.
The method according to the embodiment of the present invention is based on the above findings. That is, in the method according to the embodiment of the present invention, these impurities are incorporated into the superabsorbent polymer together with water since a superabsorbent polymer having a specific swelling ratio is used as the superabsorbent polymer. For this reason, the above-described decrease in detection sensitivity hardly occurs. As a result, it is conceived that extremely high detection sensitivity is achieved.
Further, as described above, the concentration of the sample solution and the antigen-antibody reaction proceed at the same time in the method according to the embodiment of the present invention, and thus the antigen-antibody reaction is promoted, which leads to the shortening of the test time as well.
Hereinafter, each of the steps included in the method according to the embodiment of the present invention will be described.

[Concentration step]

A concentration step is a step of mixing a solution capable of containing an antigen (a sample solution), a superabsorbent polymer, and a labeled antibody against the above antigen, to obtain a concentrated and mixed solution which is a concentrated mixed solution containing composite bodies of the above antigen and the labeled antibody.

[Sample solution]

The sample solution that is used in the concentration step is not particularly limited as long as it is a solution capable of containing an antigen. Examples of such a solution include a biological specimen, particularly a biological specimen of animal origin (particularly, of human origin) such as a body fluid (for example, blood, serum, plasma, spinal fluid, tear fluid, sweat, urine, pus, nasal mucus, or sputum), and mouthwash fluid.

The sample solution is preferably urine due to the reason that the effects and the like of the present invention are more excellent. In a case where the sample solution is urine, the concentration of urea in the concentrated and mixed solution obtained in the concentration step is preferably 5 times or less, more preferably 4.8 times or less, and still more preferably 4.6 times or less, with respect to the concentration of urea in the sample solution due to the reason that the effects and the like of the present invention are more excellent.

<Antigen>

Examples of the antigen include a fungus, a bacterium (for example, the tubercle bacillus or lipoarabinomannan (LAM) included in the tubercle bacillus), a virus (for example, an influenza virus), and a nuclear protein thereof. LAM is a major antigen in tuberculosis and a glycolipid which is a major constitutional component of the cell membrane and the cell wall. The antigen is more preferably a virus (particularly, an influenza virus) or LAM and still more preferably LAM due to the reason that the effects and the like of the present invention are more excellent.

<Pretreatment of sample solution>

Regarding the sample solution, it is possible to use a sample solution as it is or in a form of an extraction solution obtained by extracting an antigen using an appropriate solvent for extraction, in a form of a diluent solution obtained by diluting an extraction solution with an appropriate diluent, or in a form in which an extraction solution has been concentrated by an appropriate method.

As the solvent for extraction, it is possible to use a solvent (for example, water, physiological saline, and a buffer solution) that is used in a general immunological analysis method, or a water-miscible organic solvent with which a direct antigen-antibody reaction can be carried out by being diluted with such a solvent.

[Superabsorbent polymer]

The superabsorbent polymer that is used in the concentration step is a polymer having a swelling ratio of more than 0.2 g/g and less than 800 g/g (hereinafter, also referred to as a "specific superabsorbent polymer"). Here, the swelling ratio is a value defined as "a mass (g) of water held by 1 g of a superabsorbent polymer".

The specific superabsorbent polymer is not particularly limited as long as the swelling ratio thereof is more than 0.2 g/g and less than 800 g/g; however, due to the reason that the effects and the like of the present invention are more excellent, it is preferably a polyacrylic acid-based, polyacrylamide-based, cellulose-based, or polyethylene oxide-based polymer.

<Swelling ratio>

As described above, the swelling ratio of the specific superabsorbent polymer is more than 0.2 g/g and less than 800 g/g. Among the above, due to the reason that the effects and the like of the present invention are more excellent, it is preferably 1.0 g/g or more and 600 g/g or less, more preferably 10 g/g or more and 500 g/g or less, and still more preferably 20 g/g or more and 100 g/g or less.

(Method of measuring swelling ratio)

A mass of a superabsorbent polymer stored for 10 days at 25°C and 5% of relative humidity (RH) is measured, and immediately after the measurement, the superabsorbent polymer is immersed in a large amount of distilled water. After 120 minutes, the superabsorbent polymer is taken out, the water on the surface thereof is removed, the mass thereof is measured again, and the swelling ratio thereof is measured using the following expression.

$$\{(\text{Mass (g) after water absorption} - \text{initial mass (g) before water absorption})/\text{initial mass (g) before water absorption}\}$$

The method of adjusting the swelling ratio to the above-described specific range is not particularly limited; however, examples thereof include changing the kind of the polymer, changing the molecular weight of the polymer, changing the degree of crosslinking of the polymer, and changing the particle diameter of the polymer.

<Water absorption rate>

The water absorption rate of the specific superabsorbent polymer is not particularly limited; however, due to the reason that the effects and the like of the present invention are more excellent, it is preferably 0.01 g/min or more and 40 g/min or less per 1 g of superabsorbent polymer, and more preferably 0.02 g/min or more and 40 g/min or less per 1 g of superabsorbent polymer.

The water absorption rate is measured as follows.

A mass (a mass Mo, unit: g) of a superabsorbent polymer stored for 10 days at 25°C and 5% of relative humidity (RH) is measured, and immediately after the measurement, the superabsorbent polymer is immersed in a large amount of distilled water. After 10 minutes, the superabsorbent polymer is taken out, the water on the surface thereof is removed, and the mass thereof (a mass Mio) is measured. Immediately after the mass measurement, the superabsorbent polymer is immersed again in a large amount of distilled water. After 10 minutes, the superabsorbent polymer is taken out, the water on the surface thereof is removed, and the mass thereof (a mass $M_{20}$) is measured again. Immediately after the measurement of mass $M_{20}$, the superabsorbent polymer is immersed again in a large amount of distilled water. After 10 minutes, the superabsorbent polymer is taken out, the water on the surface thereof is removed, and the mass thereof (a mass $M_{30}$) is measured again.

The water absorption amount is defined as follows.

Water absorption amount for 10 minutes:

$$\Delta M10 = (M_{10} - M_0)/M_0$$

Water absorption amount for 20 minutes:

$$\Delta M20 = (M_{20} - M_0)/M_0$$

Water absorption amount for 30 minutes:

$$\Delta M30 = (M_{30} - M_0)/M_0$$

Using the water absorption amount defined as described above, the water absorption rate is calculated as follows.

Three points are plotted on the X-Y plane as the horizontal axis of time (x = 10, 20, 30, unit: minute) and the vertical axis of water absorption amount (y = $\Delta$M10, $\Delta$M20, $\Delta$M30, unit: g (water)/g (polymer amount)), to obtain a linear approximate expression of the water absorption amount with respect to the time by using the least squares method, and the slope of the linear approximate expression is defined as the water absorption rate per unit time (minute).

<Particle diameter>

The specific superabsorbent polymer preferably has a particle shape, and the particle diameter in such a case is preferably 5 mm or less, more preferably 4 mm or less, and still more preferably 3 mm or less, due to the reason that the effects and the like of the present invention are more excellent. The lower limit of the particle diameter of the specific superabsorbent polymer is preferably 0.001 mm or more, more preferably 0.005 mm or more, and still more preferably 0.01 mm or more, due to the reason that the effects and the like of the present invention are more excellent. According to a method of measuring a particle diameter of a specific superabsorbent polymer having a particle shape, the diameters of 50 particles of the polymer having a particle shape are measured with an optical microscope, and the arithmetic average value thereof can be used as the particle diameter.

<Using amount>

The using amount of the specific superabsorbent polymer is not particularly limited; however, it is preferably 0.01 to 100 g and more preferably 0.1 to 50 g with respect to 1 mL of the sample solution due to the reason that the effects and the like of the present invention are more excellent.

[Labeled antibody]

The labeled antibody used that is used in the concentration step is not particularly limited as long as it is a labeled antibody against the antigen in the above-described sample solution. Here, the labeled antibody is an antibody to which a detectable substance as a label is bound, and the label is, for example, a detectable substance and is a directly detectable substance, for example, a substance capable of generating color, fluorescence, or an electromagnetic wave such as light, or a substance capable of scattering color, fluorescence, or an electromagnetic wave such as light. Furthermore, it is a substance or state including an enzyme or the like, which forms a luminescent substance or coloring substance by interacting with a luminescent substance precursor or coloring substance precursor.

The labeled antibody is preferably an antibody capable of binding to the above-described modified antigen with a metal particle that exhibits a vivid color tone upon irradiation with an electromagnetic wave such as visible light due to the reason that the effects and the like of the present invention are more excellent. Due to the reason that the effects and the like of the present invention are more excellent, the metal particle is more preferably a gold particle. That is, the labeled antibody is preferably an antibody-modified gold particle described later due to the reason that the effects and the like of the present invention are more excellent. The labeled antibody is still more preferably an antibody-modified gold colloid particle which is a monoclonal antibody capable of binding to the above-described labeled (modified) antigen with a gold colloid particle due to the reason that the effects and the like of the present invention are more excellent.

<Antibody-modified gold particle>

The antibody-modified gold particle is a gold particle modified with an antibody (an antibody modified with a gold particle) that is capable of binding to the above-described antigen.

(Gold particle)

The gold particle is not particularly limited; however, it is preferably a gold colloid particle due to the reason that the effects and the like of the present invention are more excellent. In a case where the method according to the embodiment of the present invention includes a silver amplification step described later, the gold particle acts as a catalyst that reduces silver ions in the silver amplification step.

The particle diameter of the gold particles is preferably 500 nm or less, more preferably 300 nm or less, still more preferably 200 nm or less, and particularly preferably 150 nm or less, due to the reason that the effects and the like of the present invention are more excellent. The lower limit of the particle diameter of the gold particles is not particularly limited; however, it is preferably 5 nm or more, more preferably 7 nm or more, and still more preferably 10 nm or more, due to the reason that the effects and the like of the present invention are more excellent.

The particle diameter can be measured with a commercially available particle diameter distribution meter or the like. As a method of measuring the particle size distribution, optical microscopy, confocal laser microscopy, electron microscopy, atomic force microscopy, static light scattering method, laser diffraction method, dynamic light scattering method, centrifugal sedimentation method, electric pulse measurement method, chromatography method, ultrasonic attenuation method, and the like are known, and apparatuses corresponding to the respective principles are commercially available. As the method of measuring a particle diameter, a dynamic light scattering method can be preferably used due to the particle diameter range and the ease of measurement. Examples of the commercially available measuring device using dynamic light scattering include NANOTRAC UPA (Nikkiso Co., Ltd.), a dynamic light scattering type particle size distribution measuring device LB-550 (HORIBA, Ltd.), and a Fiber-Optics Particle Size Analyzer FPAR-1000 (Otsuka Electronics Co., Ltd.). In the present invention, the average particle diameter is obtained as a value of a median diameter (d = 50) measured at a measurement temperature of 25°C.

(Antibody)

The antibody is not particularly limited as long as it is capable of binding to the above-described antigen. However, it is possible to use, for example, an antiserum prepared from a serum of an animal immunized with an antigen, or an immunoglobulin fraction purified from an antiserum. In addition, it is possible to use a monoclonal antibody obtained by cell fusion using spleen cells of an animal immunized with an antigen, or a fragment thereof (for example, $F(ab')_2$, Fab, Fab', or Fv). The preparation of these antibodies can be carried out by a conventional method. The antibody is preferably a polyclonal antibody or a monoclonal antibody and more preferably a monoclonal antibody due to the reason that the effects and the like of the present invention are more excellent.

In a case where the antigen is LAM, examples of the antibody include the A194-01 antibody described in WO2017/139153A. The entire content disclosed in WO2017/139153A relating to the A194-01 antibody is incorporated in the present specification as a part of the disclosure of the present specification. In a case where the antigen is LAM, other examples of the antibody include the antibody having a sequence described as MoAb1 in paragraph No. [0080] of WO2013/129634A. The entire content disclosed in WO2013/129634A relating to the MoAb1 antibody is incorporated in the present specification as a part of the disclosure of the present specification.

(Method of manufacturing antibody-modified gold particle)

The method of manufacturing the antibody-modified gold particle is not particularly limited, and a known method can be used. Examples thereof include a chemical bonding method such as a method of utilizing the fact that gold and an SH group are chemically bonded and carrying out immobilization with an Au-S bond that is generated on the Au surface when an SH bond of an antibody is cleaved in a case where the SH bond thereof approaches a gold particle.

[Suitable aspect]

Due to the reason that the effects and the like of the present invention are more excellent, the concentration step is preferably a step of mixing a buffer that is capable of maintaining a pH to a constant value or a blocking agent that prevents non-specific adsorption of impurities, in addition to the above-described sample solution, the above-described specific superabsorbent polymer, and the above-described labeled antibody. The buffer is preferably tricine, and the blocking agent is preferably casein.

[Procedure of concentration step]

The procedure of the concentration step is not particularly limited; however, a method of mixing the above-described sample solution, the above-described specific superabsorbent polymer, and a pad (preferably, a gold colloid holding pad which is a pad holding the above-described antibody-modified gold colloid particles) holding the above-described labeled antibody is preferable due to the reason that the effects and the like of the present invention are more excellent. In this case, the labeled antibody is dispersed from the pad into the sample solution, and the labeled antibody reacts with the antigen to form composite bodies.

<Mixing time>

The time (mixing time) taken for mixing the above-described sample solution, the above-described specific superabsorbent polymer, and the above-described labeled antibody is not particularly limited; however, it is preferably 0.1 to 1,000 minutes, more preferably 0.1 to 800 minutes, and more preferably 0.1 to 100 minutes, due to the reason that the effects and the like of the present invention are more excellent.

[Detection step]

The detection step is a step of detecting a composite body (a composite body of an antigen and a labeled antibody) in the concentrated and mixed solution obtained in the above-described concentration step by using an antigen-antibody reaction.

The detection step is not particularly limited as long as an antigen-antibody reaction is used, and examples thereof include an enzyme immunoassay (EIA), a solid phase enzyme-linked immuno-sorbent assay (ELISA), a radioimmunoassay (RIA), a fluorescent immunoassay (FIA), a Western blot method, and immunochromatography. Among the above, immunochromatography is preferable due to the reason that the effects and the like of the present invention are more excellent. That is, the method according to the embodiment of the present invention is preferably immunochromatography.

[Suitable aspect]

Hereinafter, a suitable aspect of the method according to the present invention in a case where the detection step is immunochromatography will be described. Due to the reason that the effects and the like of the present invention are more excellent, the method according to the embodiment of the present invention preferably includes a concentration step of mixing a solution capable of containing an antigen (a sample solution), a specific superabsorbent polymer, and the above-described labeled antibody (the antibody-modified gold particle), to obtain a concentrated and mixed solution which is a concentrated mixed solution containing composite bodies (gold particle composite bodies) of an antigen, an antibody, and a gold particle, which is the composite bodies of the antigen and the labeled antibody (the antibody-modified gold particle); a spreading step of spreading the concentrated and mixed solution obtained in the concentration step on an insoluble carrier having a reaction site at which a binding substance capable of binding to the above-described antigen is immobilized; and a capturing step of capturing the gold particle composite bodies at the reaction site of the insoluble carrier. Among the above, due to the reason that the effects and the like of the present invention are more excellent, it preferably further includes a silver amplification step of silver-amplifying the gold particle composite bodies captured in the capturing step.

Hereinafter, each of the steps included in the above suitable aspect will be described.

[Concentration step]

As described above, the concentration step is a step of mixing a solution capable of containing an antigen (a sample solution), a specific superabsorbent polymer, and the above-described labeled antibody (the antibody-modified gold particle), to obtain a concentrated and mixed solution which is a concentrated mixed solution containing gold particle composite bodies which are the composite bodies of the antigen and the labeled antibody (the antibody-modified gold particle). The sample solution, the specific superabsorbent polymer, and the antibody-modified gold particle are as described above.

[Spreading step]

As described above, the spreading step is a step of spreading the concentrated and mixed solution obtained in the above-described concentration step on an insoluble carrier having a reaction site at which a binding substance capable of binding to the above-described antigen is immobilized. As a result, the gold particle composite bodies in the concentrated and mixed solution are spread on the insoluble carrier.

<Insoluble carrier>

The above-described insoluble carrier is an insoluble carrier having a reaction site (a test line) at which a binding substance capable of binding to the above-described antigen is immobilized. The insoluble carrier may have a plurality of test lines depending on the kinds of antigens (for example, a test line for influenza A type virus and a test line for influenza B type virus). In addition, the insoluble carrier may have a control line on the downstream side of the test line in order to check the spreading of the gold particle composite bodies. Further, in a case where the method according to the embodiment of the present invention includes a silver amplification step described later and in a case where a reducing agent solution is used in the silver amplification step described later, a coloring reagent immobilization line may be provided on the downstream side of the test line in order to detect the reducing agent solution.

Examples of the specific aspect of the insoluble carrier include a nitrocellulose membrane 100 as illustrated in Fig. 1, which has from the upstream side; an addition pad 1, a test line 2, a control line 3, and a coloring reagent immobilization line 4. Here, the addition pad 1 is a pad to which the concentrated and mixed solution is added, the test line 2 is a line on which the binding substance is immobilized, the control line 3 is a line for checking the spreading, and the coloring reagent immobilization line 4 is a line for detecting the reducing agent solution described later. Here, the upstream side and the downstream side mean descriptions intended to indicate the spreading from the upstream side to the downstream side at the time when gold particle composite bodies are spread.

The more specific aspect of the insoluble carrier (or an immunochromatographic kit having the insoluble carrier) include, for example, the insoluble carrier or the immunochromatographic kit disclosed in JP5728453B, and the entire content of JP5728453B relating to the insoluble carrier and the immunochromatographic kit is incorporated in the present specification as a part of the disclosure of the present specification.

(Insoluble carrier)

The insoluble carrier is preferably a porous carrier. In particular, it is preferably a nitrocellulose film (a nitrocellulose membrane), a cellulose membrane, an acetyl cellulose membrane, a polysulfone membrane, a polyether sulfone membrane, a nylon membrane, a glass fiber, a non-woven fabric, a cloth, a thread, or the like, and more preferably a nitrocellulose film, due to the reason that the effects and the like of the present invention are more excellent.

(Binding substance)

The binding substance is not particularly limited as long as it is capable of binding to the above-described antigen. Due to the reason that the effects and the like of the present invention are more excellent, the binding substance is preferably a protein, more preferably an antibody (for example, a polyclonal antibody or a monoclonal antibody), and from the viewpoint of achieving higher detection sensitivity, it is still more preferably a monoclonal antibody. Specific examples and the preferred aspect in a case where the binding substance is an antibody are respectively the same as those for the antibody of the above-described labeled antibody.

The binding substance may be the same as or different from the antibody of the above-described labeled antibody; however, an aspect in which the second binding substance is a different substance is preferable due to the reason that the effects and the like of the present invention are more excellent. In addition, in a case where the binding substance is an antibody, the antibody of the above-described labeled antibody and the above-described binding substance as an antibody are preferably different from each other due to the reason that the effects and the like of the present invention are more excellent. Further, in a case where the binding substance is an antibody, an aspect in which an epitope (a part of an antigen recognized by an antibody) of the labeled antibody and an epitope (a part of an antigen recognized by an antibody) of the binding substance are different from each other is preferable due to the reason that the effects and the like of the present invention are more excellent. The difference in epitope between antibodies can be confirmed by, for example, an enzyme-linked immuno-sorbent assay (ELISA).

<Spreading>

The method of spreading the concentrated and mixed solution on the insoluble carrier is not particularly limited; however, examples thereof include a method in which the above nitrocellulose membrane 100 (or an immunochromatographic kit having the nitrocellulose membrane 100) as illustrated in Fig. 1 is prepared, and the concentrated and mixed solution is dropwise added onto an addition pad and moved from the upstream side to the downstream side by using the capillary action as illustrated in Fig. 1.

[Capturing step]

The capturing step is a step of capturing the gold particle composite bodies at the reaction site of the insoluble carrier. As described above, since the binding substance capable of binding to an antigen is immobilized at the reaction site of the insoluble carrier, the gold particle composite bodies (the composite bodies of an antigen and an antibody-modified gold particle) that have been spread on the insoluble carrier in the spreading step are captured at the reaction site (the test line) of the insoluble carrier. The captured gold particle composite bodies are visible since it is colored by the surface plasmon or the like of a gold particle. In addition, it is also possible to estimate the concentration of the captured composite bodies using an image analysis device or the like. In this way, the antigen in the specimen can be detected. In a case where a specimen does not contain an antigen, the gold particle composite bodies are not formed, and thus it is not captured at the reaction site of the insoluble carrier and coloration does not occur.

[Silver amplification step]

The silver amplification step is a step of silver-amplifying the gold particle composite bodies captured in the capturing step. The silver amplification step is a step of forming large silver particles in the gold particle composite bodies captured at the reaction site of the insoluble carrier by providing silver ions to the insoluble carrier after the capturing step. More specifically, it is a step in which silver ions are reduced using gold particles of the gold particle composite bodies as a catalyst to form silver particles (for example, a diameter of 10 $\mu$m or more). This significantly improves the detection sensitivity of the captured gold particle composite bodies.

<Suitable aspect>

The method of providing silver ions to the insoluble carrier after the capturing step is not particularly limited; however, it is preferably a method in which the following reducing agent solution and the following silver amplification solution are used, due to the reason that the effects and the like of the present invention are more excellent. Further, in addition to the reducing agent solution and the silver amplification solution, a washing solution may be used to wash the gold particle composite bodies remaining on the insoluble carrier except for the specific binding reaction. The reducing agent solution may also serve as a washing solution.

(Reducing agent solution)

The reducing agent solution contains a reducing agent capable of reducing silver ions. As the reducing agent capable of reducing silver ions, any inorganic or organic material or a mixture thereof can be used as long as it can reduce silver ions to silver. Preferred examples of the inorganic reducing agent include a reducing metal salt and a reducing metal composite body salt, of which the atomic valence is capable of being changed with a metal ion such as $Fe^{2+}$, $V^{2+}$, or $Ti^{3+}$. In a case where an inorganic reducing agent is used, it is necessary to remove or detoxify oxidized ions by chelating or reducing the oxidized ions. For example, in a system in which $Fe^{2+}$ is used as the reducing agent, a complex of $Fe^{3+}$, which is an oxide, is formed using citric acid or ethylenediaminetetraacetic acid (EDTA), and therefore detoxification is possible. In the present invention, it is preferable to use such an inorganic reducing agent, and as a more preferred aspect of the present invention, it is preferable to use a metal salt of $Fe^{2+}$ as the reducing agent.

It is also possible to use, as the reducing agent, a main developing agent (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or derivatives thereof), or leuco dyes) that is used in a wet-type light-sensitive silver halide photographic material, and other materials obvious to those who are skilled in the technology in the present field, such as a material disclosed in US6020117A.

As the reducing agent, an ascorbic acid reducing agent is also preferable. The useful ascorbic acid reducing agent includes ascorbic acid, an analogous substance thereof, an isomer thereof, and a derivative thereof. Preferred examples thereof include D- or L-ascorbic acid and a sugar derivative thereof (for example, γ-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly preferred examples thereof include D-, L-, or D,L-ascorbic acid (and an alkali metal salt thereof) or isoascorbic acid (or an alkali metal salt thereof), and a sodium salt is a preferred salt. A mixture of these reducing agents can be used as necessary.

Due to the reason that the effects and the like of the present invention are more excellent, the reducing agent solution is preferably allowed to flow so that the angle between the spreading direction in the spreading step and the spreading direction of the reducing agent solution is 0 degrees to 150 degrees, and more preferably allowed to flow so that the angle between the spreading direction in the spreading step and the spreading direction of the reducing agent solution is 0 degrees to 135 degrees. Examples of the method of regulating the angle between the spreading direction in the spreading step and the spreading direction of the reducing agent solution include the method described in Examples of JP2009-150869A.

(Silver amplification solution)

The silver amplification solution is a solution containing a compound containing silver ions. As the compound containing silver ions, it is possible to use, for example, an organic silver salt, an inorganic silver salt, or a silver complex. Preferred examples thereof include silver ion-containing compounds having a high solubility in a solvent such as water, such as silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

The organic silver salt, the inorganic silver salt, or the silver complex is contained as the silver in the silver amplification solution at a concentration of 0.001 mol/L to 5 mol/L, preferably 0.005 mol/L to 3 mol/L, and more preferably 0.01 mol/L to 1 mol/L.

Examples of the auxiliary agent of the silver amplification solution include a buffer, a preservative such as an antioxidant or an organic stabilizer, and a rate regulating agent. As the buffer, it is possible to use, for example, a buffer formed of acetic acid, citric acid, sodium hydroxide, or one of salts of these compounds, or formed of tris(hydroxymethyl)aminomethane, or other buffers that are used in general chemical experiments. These buffers are appropriately used to adjust the pH of the amplification solution to an optimum pH thereof. In addition, as the antifogging agent, an alkyl amine can be used as an auxiliary agent, and a dodecyl amine is particularly preferable. In addition, a surfactant can be used for the intended purpose of improving the solubility of this auxiliary agent, and $C_9H_{19}$-$C_6H_4$-O-$(CH_2CH_2O)_{50}$H is particularly preferable.

Due to the reason that the effects and the like of the present invention are more excellent, the silver amplification solution is preferably allowed to flow from the direction opposite to the spreading direction in the spreading step described above and more preferably allowed to flow so that the angle between the spreading direction in the spreading step and the spreading direction of the silver amplification solution is 45 degrees to 180 degrees. Examples of the method of regulating the angle between the spreading direction in the spreading step and the spreading direction of the silver amplification solution include the method described in Examples of JP2009-150869A. Further, a supply aspect in which the silver amplification solution is added dropwise onto the insoluble carrier from above is also preferable in order to carry out silver amplification in a short time.

Examples

Hereinafter, the present invention will be described in more detail with reference to Examples; however, the present invention is not limited thereto.

[A] Example in which antigen is influenza virus

[Preparation of sample solution]

A sample solution (a solution capable of containing an antigen) was prepared using a Quick S-Influ A·B "SEIKEN" negative/positive control solution (product number: 322968, manufactured by DENKA SEIKEN Co., Ltd.). Specifically, the above positive control solution was subjected to serial 10-time dilutions with a phosphate buffered salts (PBS) buffer containing 0.1% by mass bovine serum albumin (BSA), and sample solutions (solutions capable of containing an antigen) with the respective dilution rates were prepared.

[Production of gold colloid holding pad]

The pH was adjusted by adding 1 mL of a 50 mmol/L $KH_2PO_4$ buffer (pH 7.5) to 9 mL of a solution (product number: EM. GC50, manufactured by BBI Solutions) containing gold colloid particles (particle diameter: 50 nm). 1 mL of a 160 $\mu$g/mL anti-influenza A type monoclonal antibody (Anti-influenza A SPTN-5 7307, manufactured by Medix Biochemica) was added to the above solution of which the pH had been adjusted, and stirring was carried out. After allowing to stand for 10 minutes, 550 $\mu$L of an aqueous solution of 1% polyethylene glycol ((PEG), weight-average molecular weight (Mw.): 20,000, product number: 168-11285, manufactured by FUJIFILM Wako Pure Chemical Corporation) was added thereto, stirring was carried out, and subsequently 1.1 mL of an aqueous solution of 10% bovine serum albumin ((BSA), Fraction V, product number: A-7906, manufactured by Sigma-Aldrich Co., LLC) was added thereto, and stirring was carried out. After centrifuging this solution at a centrifugal acceleration of 8,000 × g at 4°C for 30 minutes (himac CF16RX, manufactured by Hitachi, Ltd.), the supernatant was removed with about 1 mL thereof remaining, and the gold colloid particles were re-dispersed by an ultrasonic washer. Thereafter, after dispersing them in 20 mL of a gold colloid preservative solution (a 20 mmol/L Tris-HCl buffer (pH 8.2), 0.05% PEG (Mw.: 20,000), 150 mmol/L NaCl, 1% BSA) and centrifuged again at 8,000 × g and 4°C for 30 minutes, the supernatant was removed with about 1 mL thereof remaining, and the gold colloid particles were re-dispersed by an ultrasonic washer, thereby obtaining a solution of antibody-modified gold colloid particles (a labeled antibody) which are gold colloid particles (particle diameter: 50 nm) modified with the anti-influenza A type monoclonal antibody. After adjusting the concentration of the obtained solution with a buffer, the solution was added dropwise onto a 5 mm × 30 cm glass fiber pad (GFDX203000 manufactured by Merck KGaA), subsequently dried with a vacuum dryer for 15 hours, and the pad was cut to obtain a pad (a gold colloid holding pad) (5 mm × 4 mm) holding the antibody-modified gold colloid particles (the labeled antibody) which are the anti-influenza A type monoclonal antibody modified with gold colloid particles.

[Example A1]

Immunochromatography of Example A1 was carried out as follows.

[Concentration step]

2mL of the sample solution described above, 1 g of the following superabsorbent polymer, 6.7 $\mu$g of casein (manufactured by FUJIFILM Wako Pure Chemical Corporation), and 0.1 $\mu$g of tricine (manufactured by Dojindo Molecular Technologies. Inc.), one gold colloid holding pad were mixed, stirred, and allowed to stand for 20 minutes (mixing time: 20 minutes). As a result, the concentration of the sample solution and the antigen-antibody reaction proceeded at the same time, and thus gold particle composite bodies, which are the composite bodies of the influenza A type virus and the antibody-modified gold colloid particle (the labeled antibody) which is the anti-influenza A type monoclonal antibody modified with gold colloid particles, were formed in a concentrated state. The solution that had not been absorbed by the superabsorbent polymer (the concentrated and mixed solution containing the gold particle composite bodies, which is a concentrated mixed solution) was recovered with a pipette (manufactured by Eppendorf).

<Superabsorbent polymer used in Example A1>

Commercially available superabsorbent polymer (SAP) particles (model number: 197-12451, manufactured by FUJIFILM Wako Pure Chemical Corporation) were graded to obtain a superabsorbent polymer that would be used in the concentration step. The superabsorbent polymer had a particle diameter of 0.05 mm, a swelling ratio of 600 g/g, and a water absorption rate of 20 g/min.

[Spreading step]

The nitrocellulose membrane 100 as illustrated in Fig. 1 was prepared, which has from the upstream side; the addition pad 1, the test line 2, the control line 3, and the coloring reagent immobilization line 4. It is noted that the addition pad 1 is a pad to which the concentrated and mixed solution has been added; the test line 2 is a line on which the anti-influenza A type monoclonal antibody (Anti-influenza A SPTN-5 7307, manufactured by Medix Biochemica) has been immobilized; the control line 3 is a line for checking the spreading, and it is a line coated with an anti-mouse IgG antibody (Anti-mouse IgG (H + L), rabbit F(ab')$_2$, product number: 566-70621, manufactured by FUJIFILM Wako Pure Chemical Corporation); and the coloring reagent immobilization line 4 is a line for detecting the reducing agent solution described later, and it is a line coated with bromocresol green (manufactured by FUJIFILM Wako Pure Chemical Corporation).
The concentrated and mixed solution was added dropwise onto the addition pad and spread on the downstream side of the nitrocellulose membrane.

[Capturing step]

The gold particle composite bodies in the concentrated and mixed solution, which had been spread in the spreading step, were captured on the test line.

[Silver amplification step]

The silver amplification step was carried out as follows.

<Preparation of reducing agent solution>

23.6 mL of an aqueous solution of 1 mol/L iron nitrate, which was produced by dissolving iron (III) nitrate nonahydrate (manufactured by FUJIFILM Wako Pure Chemical Corporation) in water, and 13.1 g of citric acid (manufactured by FUJIFILM Wako Pure Chemical Corporation) were dissolved in 290 g of water. After all of the substances were dissolved, 36 mL of nitric acid (10% by mass) was added thereto while stirring with a stirrer, 60.8 g of ammonium iron (II) sulfate hexahydrate (manufactured by FUJIFILM Wako Pure Chemical Corporation) was added thereto, and the resultant solution was used as the reducing agent solution.

<Preparation of silver amplification solution>

8 mL of a silver nitrate solution (including 10 g of silver nitrate) and 24 mL of an aqueous solution of 1 mol/L iron nitrate were added to 66 g of water. Further, this solution was mixed with a solution obtained by dissolving in advance 5.9 mL of nitric acid (10% by mass), 0.1 g of dodecyl amine (manufactured by FUJIFILM Wako Pure Chemical Corporation), and 0.1 g of a surfactant $C_{12}H_{25}$-$C_6H_4$-O-$(CH_2CH_2O)_{50}$H in 47.6 g of water, and the resultant solution was used as the silver amplification solution.

<Spreading of reducing agent solution>

In the nitrocellulose membrane, the reducing agent solution prepared as described above was allowed to flow from the same direction as that of the spreading step described above (from the upstream side).

<Spreading of silver amplification solution>

After the coloring reagent immobilization line was discolored, the silver amplification solution prepared as described above was allowed to flow from the direction opposite to the spreading direction (from the downstream side) in the spreading step. In this way, the gold particle composite bodies captured on the test line were silver-amplified.

[Evaluation]

The coloration of the test line was visually checked, and the lowest dilution rate (the minimum detection sensitivity) at which coloration was confirmed was investigated. The results are shown in Table 1. It means that as the minimum detection sensitivity is lower, an antigen can be detected even in a specimen having a low antigen concentration, which means the detection sensitivity is high.

[Example A2]

Immunochromatography was carried out and evaluated according to the same procedure as in Example A1, except that in the concentration step, the following superabsorbent polymer was used. The results are shown in Table 1.

[Superabsorbent polymer used in Example A2]

Commercially available superabsorbent polymer (SAP) particles (manufactured by M2 Polymer Technologies Inc.; SAP Sphere 2.5 mm) were graded to obtain a superabsorbent polymer that would be used in the concentration step. The superabsorbent polymer had a particle diameter of 2.5 mm, a swelling ratio of 13 g/g, and a water absorption rate of 0.5 g/min.

[Example A3]

Immunochromatography was carried out and evaluated according to the same procedure as in Example A1, except that in the concentration step, the following superabsorbent polymer was used. The results are shown in Table 1.

[Superabsorbent polymer used in Example A3]

Superabsorbent polymer (SAP) particles were prepared with reference to the methods of Example 1 and Comparative Example 1 disclosed in JP2015-536375A, and for particles having a different particle diameter, classification was carried out using a 0.5 mm sieve to obtain superabsorbent polymer particles having a particle diameter of 0.5 mm and a swelling ratio of 30 g/g.

[Example A4]

Immunochromatography was carried out and evaluated according to the same procedure as in Example A2, except that the mixing time was changed to 40 minutes. The results are shown in Table 1.

[Comparative Example A1]

Immunochromatography was carried out and evaluated according to the same procedure as in Example A1, except that without carrying out the concentration step, the following mixed solution was used instead of the concentrated and mixed solution in the spreading step. The results are shown in Table 1.

[Mixed solution used in Comparative Example A1]

2 mL of the sample solution described above and one gold colloid holding pad described above were mixed, stirred, and allowed to stand for 40 minutes (mixing time: 40 minutes). As a result, gold particle composite bodies, which are the composite bodies of the influenza A type virus and the antibody-modified gold colloid particle (the labeled antibody) which is the anti-influenza A type monoclonal antibody modified with gold colloid particles, were formed. The mixed solution containing the gold particle composite bodies was recovered with a pipette (manufactured by Eppendorf).

[Comparative Example A2]

Immunochromatography was carried out and evaluated according to the same procedure as in Example A1, except that in the concentration step, the following superabsorbent polymer was used. As a result, coloration could not be confirmed in Comparative Example A2. It is presumed that the swelling ratio was too high and thus the concentration was not carried well.

[Superabsorbent polymer used in Comparative Example A2]

Polyacrylic acid 5000 (manufactured by FUJIFILM Wako Pure Chemical Corporation) was graded to obtain a superabsorbent polymer that would be used in the concentration step. The superabsorbent polymer had a particle diameter of 0.01 mm, a swelling ratio of 1,000 g/g, and a water absorption rate of 60 g/min.

[Comparative Example A3]

Immunochromatography was carried out and evaluated according to the same procedure as in Example A1, except that in the concentration step, the following superabsorbent polymer was used. The results are shown in Table 1.

[Superabsorbent polymer used in Comparative Example A3]

A polyvinyl alcohol (PVA) solution of 10% by mass was prepared and this solution was dropwise added to 1M HCl to prepare particles. The obtained particles were graded and used as the superabsorbent polymer that would be used in the concentration step. The superabsorbent polymer had a particle diameter of 0.5 mm, a swelling ratio of 0.2 g/g, and a water absorption rate of 0.01 g/min.

[Table 1]

| | Example A1 | Example A2 | Example A3 | Example A4 | Comparative Example A1 | Comparative Example A2 | Comparative Example A3 |
|---|---|---|---|---|---|---|---|
| Concentration step | Present | Present | Present | Present | Absent | Present | Present |
| Mixing time [minute] | 20 | 20 | 20 | 40 | 40 | 40 | 40 |
| Swelling ratio [g/g] | 600 | 13 | 30 | 13 | - | 1,000 | 0.2 |
| Particle diameter [mm] | 0.05 | 2.5 | 0.5 | 2.5 | - | 0.01 | 0.5 |
| Minimum detection sensitivity | 1/2560 | 1/1600 | 1/3200 | 1/3200 | 1/320 | - | 1/320 |

EP 4 099 017 A1

As can be seen from Table 1 and the like, as compared with Comparative Example A1 in which the concentration step was not carried out and Comparative Examples A2 and A3 in which the concentration step was carried out using a superabsorbent polymer of which the swelling ratio was out of a specific range, Examples A1 to A4 in which the concentration step was carried out using a superabsorbent polymer of which the swelling ratio was in the specific range (a specific superabsorbent polymer) exhibited high detection sensitivity.

From the comparison between Examples A1 to A3 (the comparison between aspects in which only the kind of superabsorbent polymer is different), it was seen that Examples A1 and A3 in which the swelling ratio of the specific superabsorbent polymer is 20 g/g or more exhibit high detection sensitivity. Among them, Example A3 in which the swelling ratio of the specific superabsorbent polymer was 500 g/g or less exhibited further higher detection sensitivity.

From the comparison between Examples A2 and A4 (the comparison between aspects in which only mixing time is different), it was seen that Example A4 in which the mixing time in the concentration step is 30 minutes or more exhibits higher detection sensitivity.

[B] Case where antigen is LAM

[Preparation of sample solution]

Lipoarabinomannan (LAM) (02249-61, Nacalai Tesque, Inc.) extracted from the tubercle bacillus was added to a urine sample obtained by pooling urine samples (BioIVT LLC) of healthy subjects to prepare a sample solution (a solution capable of containing an antigen) of a LAM concentration shown in Table 2.

[Production of gold colloid holding pad]

The pH was adjusted by adding 1 mL of a 50 mmol/L $KH_2PO_4$ buffer (pH 8.0) to 9 mL of a solution (product number: EM. GC50, manufactured by BBI Solutions) containing gold colloid particles (particle diameter: 50 nm). 1 mL of a 20 $\mu$g/mL anti-lipoarabinomannan (LAM) monoclonal antibody (manufactured by Nacalai Tesque, Inc., product code: 05494-84) was added to the above solution of which the pH had been adjusted, and stirring was carried out for 10 minutes. Then, after allowing to stand for 10 minutes, 550 $\mu$L of an aqueous solution containing 1% by mass of polyethylene glycol ((PEG), weight-average molecular weight (Mw.): 20,000, product number: 168-11285, manufactured by FUJIFILM Wako Pure Chemical Corporation) was added thereto, stirring was carried out for 10 minutes, and subsequently 1.1 mL of an aqueous solution of 10% by mass of bovine serum albumin ((BSA), Fraction V, product number: A-7906, manufactured by Sigma-Aldrich Co., LLC) was added thereto, and stirring was carried out for 10 minutes. This solution was subjected to centrifugal separation for 30 minutes under conditions of 8,000 $\times$ g at 4°C using a centrifugal separator (himac CF16RX, manufactured by Hitachi, Ltd.). The supernatant solution was removed with 1 mL thereof remaining at the bottom of a container, and gold colloid particles contained in the 1 mL solution remaining at the bottom of the container were re-dispersed by an ultrasonic washer. Thereafter, they were dispersed in 20 mL of a gold colloid preservative solution (a 20 mmol/L Tris-HCl buffer (pH 8.2), 0.05% PEG (Mw.: 20,000), 150 mmol/L NaCl, 1% BSA), centrifugal separation was carried out again using the same centrifugal separator under the same conditions, the supernatant solution was removed, and after ultrasonic dispersion, they were dispersed in the gold colloid preservative solution, thereby obtaining a solution of antibody-modified gold colloid particles (a labeled antibody) which are gold colloid particles (particle diameter: 50 nm) modified with the anti-LAM monoclonal antibody. After adjusting the concentration of the obtained solution with a buffer, the solution was added dropwise onto a 5 mm $\times$ 30 cm glass fiber pad (GFDX203000 manufactured by Merck KGaA), subsequently dried with a vacuum dryer for 15 hours, and the pad was cut to obtain a pad (a gold colloid holding pad) (5 mm $\times$ 4 mm) holding the antibody-modified gold colloid particles (the labeled antibody) which are gold colloid particles modified with the anti-LAM monoclonal antibody.

[Example B1]

Immunochromatography of Example B1 was carried out as follows.

[Concentration step]

2mL of the sample solution described above, 1 g of the superabsorbent polymer that is the same as the above-described superabsorbent polymer used in Example A1, 6.7 $\mu$g of casein (manufactured by FUJIFILM Wako Pure Chemical Corporation), and 0.1 $\mu$g of tricine (manufactured by Dojindo Molecular Technologies. Inc.), one gold colloid holding pad were mixed, stirred, and allowed to stand for 20 minutes (mixing time: 20 minutes). As a result, the concentration of the sample solution and the antigen-antibody reaction proceeded at the same time, and thus gold particle composite bodies, which are the composite bodies of the LAM and the antibody-modified gold colloid particle (the labeled antibody) which is the anti-LAM monoclonal antibody modified with gold colloid particles, were formed in a concentrated state. The solution that had not been absorbed by the superabsorbent polymer (the concentrated and mixed solution containing the gold particle composite bodies, which is a concentrated mixed solution) was recovered with a pipette (manufactured by Eppendorf). The concentration of urea in the concentrated and mixed solution was 5 times or less with respect to the concentration of urea in the sample solution.

[Spreading step]

The nitrocellulose membrane 100 as illustrated in Fig. 1 was prepared, which has from the upstream side; the addition pad 1, the test line 2, the control line 3, and the coloring reagent immobilization line 4. It is noted that the addition pad 1 is a pad to which the concentrated and mixed solution is added, the test line 2 is a line on which the anti-LAM monoclonal antibody is immobilized, the control line 3 is a line for checking the spreading, and the coloring reagent immobilization line 4 is a line for detecting the reducing agent solution of the silver amplification step described later.
The concentrated and mixed solution was added dropwise onto the addition pad and spread on the downstream side of the nitrocellulose membrane.

[Capturing step]

The gold particle composite bodies in the concentrated and mixed solution, which had been spread in the spreading step, were captured on the test line.

[Silver amplification step]

The silver amplification step was carried out according to the same procedure as in Example A1.
In this way, the gold particle composite bodies captured on the test line were silver-amplified.

[Evaluation]

The coloration of the test line was visually checked and evaluated according to the following criteria.

++: The coloration is observed.
+: The coloration is weak.
-: No coloration is observed.

The results are shown in Table 2. It means that the lower the lowest LAM concentration (the minimum detection sensitivity) among the LAM concentrations of the sample solution evaluated as ++ or +, the higher the detection sensitivity.

[Example B2]

Immunochromatography was carried out and evaluated according to the same procedure as in Example B1, except that in the concentration step, the same superabsorbent polymer as the superabsorbent polymer used in Example A2 described above was used. The results are shown in Table 2. The concentration of urea in the concentrated and mixed solution was 5 times or less with respect to the concentration of urea in the sample solution.

[Example B3]

Immunochromatography was carried out and evaluated according to the same procedure as in Example B1, except that in the concentration step, the same superabsorbent polymer as the superabsorbent polymer used in Example A3 described above was used. The results are shown in Table 2. The concentration of urea in the concentrated and mixed solution was 5 times or less with respect to the concentration of urea in the sample solution.

[Example B4]

Immunochromatography was carried out and evaluated according to the same procedure as in Example B2, except that the mixing time was changed to 40 minutes. The results are shown in Table 2. The concentration of urea in the concentrated and mixed solution was 5 times or less with respect to the concentration of urea in the sample solution.

[Comparative Example B1]

Immunochromatography was carried out and evaluated according to the same procedure as in Example B1, except that without carrying out the concentration step, the following mixed solution was used instead of the concentrated and mixed solution in the spreading step. The results are shown in Table 2.

[Mixed solution used in Comparative Example B1]

2 mL of the sample solution described above and one gold colloid holding pad described above were mixed, stirred, and allowed to stand for 40 minutes (mixing time: 40 minutes). As a result, gold particle composite bodies, which are composite bodies of the LAM and the antibody-modified gold colloid particle (the labeled antibody) which is a gold colloid particle modified with the anti-LAM monoclonal antibody, were formed. The mixed solution containing the gold particle composite bodies was recovered with a pipette (manufactured by Eppendorf).

[Comparative Example B2]

Immunochromatography was carried out and evaluated according to the same procedure as in Example B1, except that in the concentration step, the same superabsorbent polymer as the superabsorbent polymer used in Comparative Example A2 described above was used. The results are shown in Table 2. The coloration could not be confirmed in Comparative Example B2. It is presumed that the swelling ratio was too high and thus the concentration was not carried well.

[Comparative Example B3]

Immunochromatography was carried out and evaluated according to the same procedure as in Example B1, except that in the concentration step, the same superabsorbent polymer as the superabsorbent polymer used in Comparative Example A3 described above was used. The results are shown in Table 2.

[Table 2]

| | | Example B1 | Example B2 | Example B3 | Example B4 | Comparative Example B1 | Comparative Example B2 | Comparative Example B3 |
|---|---|---|---|---|---|---|---|---|
| Concentration step | | Present | Present | Present | Present | Absent | Present | Present |
| Mixing time [minute] | | 20 | 20 | 20 | 40 | 40 | 40 | 40 |
| Swelling ratio [g/g] | | 600 | 13 | 30 | 13 | - | 1,000 | 0.2 |
| Particle diameter [mm] | | 0.05 | 2.5 | 0.5 | 2.5 | - | 0.01 | 0.5 |
| LAM concentration [ng/mL] | 0.2 | ++ | ++ | ++ | ++ | ++ | - | ++ |
| | 0.1 | ++ | ++ | ++ | ++ | ++ | - | + |
| | 0.05 | ++ | ++ | ++ | ++ | + | - | + |
| | 0.025 | ++ | ++ | ++ | ++ | - | - | - |
| | 0.0125 | + | + | ++ | ++ | - | - | - |
| | 0.01 | + | + | + | ++ | - | - | - |
| | 0.005 | + | - | + | + | - | - | - |
| | 0.0025 | - | - | + | - | - | - | - |

As can be seen from Table 2 and the like, as compared with Comparative Example B1 in which the concentration step was not carried out and Comparative Examples B2 and B3 in which the concentration step was carried out using a superabsorbent polymer of which the swelling ratio was out of a specific range, Examples B1 to B4 in which the concentration step was carried out using a superabsorbent polymer of which the swelling ratio was in the specific range (a specific superabsorbent polymer) exhibited high detection sensitivity.

From the comparison between Examples B1 to B3 (the comparison between aspects in which only the kind of superabsorbent polymer is different), it was seen that Examples B1 and B3 in which the swelling ratio of the specific superabsorbent polymer is 20 g/g or more exhibit high detection sensitivity. Among them, Example B3 in which the swelling ratio of the specific superabsorbent polymer was 500 g/g or less exhibited further higher detection sensitivity.

From the comparison between Examples B2 and B4 (the comparison between aspects in which only mixing time is different), it was seen that Example B4 in which the mixing time in the concentration step is 30 minutes or more exhibits higher detection sensitivity.

Explanation of References

1: addition pad
2: test line
3: control line
4: coloring reagent immobilization line
100: nitrocellulose membrane

**Claims**

1. An immunological test method, comprising:

    a concentration step of mixing a solution capable of containing an antigen, a superabsorbent polymer, and a labeled antibody against the antigen, to obtain a concentrated and mixed solution which is a concentrated mixed solution containing composite bodies of the antigen and the labeled antibody; and
    a detection step of detecting the composite bodies in the concentrated and mixed solution using an antigen-antibody reaction,
    wherein a swelling ratio of the superabsorbent polymer is more than 0.2 g/g and less than 800 g/g.

2. The immunological test method according to claim 1, wherein the solution capable of containing an antigen is urine.

3. The immunological test method according to claim 2, wherein a concentration of urea in the concentrated and mixed solution is 5 times or less with respect to a concentration of urea in the solution capable of containing an antigen.

4. The immunological test method according to any one of claims 1 to 3, wherein a water absorption rate of the superabsorbent polymer is 0.01 g/min or more and 40 g/min or less per 1 g of the superabsorbent polymer.

5. The immunological test method according to any one of claims 1 to 4, wherein the immunological test method is immunochromatography.

6. The immunological test method according to any one of claims 1 to 5, wherein the labeled antibody is an antibody-modified gold colloid particle which is a monoclonal antibody capable of binding to the antigen labeled with a gold colloid particle.

7. The immunological test method according to any one of claims 1 to 6, wherein the superabsorbent polymer is a polyacrylic acid-based, polyacrylamide-based, cellulose-based, or polyethylene oxide-based polymer.

8. The immunological test method according to any one of claims 1 to 7, wherein a particle diameter of the superabsorbent polymer is 5 mm or less and 0.001 mm or more.

9. The immunological test method according to any one of claims 1 to 8, wherein the concentration step is a step of mixing casein and tricine in addition to the solution capable of containing an antigen, the superabsorbent polymer, and the labeled antibody.

FIG. 1

**INTERNATIONAL SEARCH REPORT**

| International application No. |
|---|
| PCT/JP2020/048659 |

**A. CLASSIFICATION OF SUBJECT MATTER**
Int.Cl. G01N33/531(2006.01)i, G01N33/543(2006.01)i
FI: G01N33/543521, G01N33/531B, G01N33/543541Z, G01N33/543501M

According to International Patent Classification (IPC) or to both national classification and IPC

**B. FIELDS SEARCHED**

Minimum documentation searched (classification system followed by classification symbols)
Int.Cl. G01N33/531, G01N33/543

Documentation searched other than minimum documentation to the extent that such documents are included in the fields searched
Published examined utility model applications of Japan    1922-1996
Published unexamined utility model applications of Japan    1971-2021
Registered utility model specifications of Japan    1996-2021
Published registered utility model applications of Japan    1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2008-506930 A (UMEDIK INC.) 06 March 2008 (2008-03-06), paragraphs [0001]-[0030], fig. 2 | 1-9 |
| Y | JP 2000-72822 A (HYMO CORPORATION) 07 March 2000 (2000-03-07), paragraphs [0001], [0015] | 1-9 |
| A | JP 2013-543110 A (QIAGEN GMBH) 28 November 2013 (2013-11-28), entire text, all drawings | 1-9 |
| A | WO 2005/058453 A1 (PREENTEC AG) 30 June 2005 (2005-06-30), entire text, all drawings | 1-9 |
| A | US 2019/0105653 A1 (THE REGENTS OF THE UNIVERSITY OF CALIFORNIA) 11 April 2019 (2019-04-11), entire text, all drawings | 1-9 |

☒ Further documents are listed in the continuation of Box C.     ☒ See patent family annex.

| | |
|---|---|
| *   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 04 March 2021 | 16 March 2021 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

EP 4 099 017 A1

**INTERNATIONAL SEARCH REPORT**

| International application No. |
|---|
| PCT/JP2020/048659 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 2008-249422 A (FUJIFILM CORPORATION) 16 October 2008 (2008-10-16), entire text, all drawings | 1-9 |

Form PCT/ISA/210 (second sheet) (January 2015)

24

| | | INTERNATIONAL SEARCH REPORT | International application No. |
|---|---|---|---|
| | | Information on patent family members | PCT/JP2020/048659 |

| JP 2008-506930 A | 06 March 2008 | WO 2006/007711 A1 pages 1-9, fig. 2 EP 1774277 A CA 2474940 A1 CA 2573925 A1 CN 101052866 A AU 2005263153 A |
|---|---|---|
| JP 2000-72822 A | 07 March 2000 | (Family: none) |
| JP 2013-543110 A | 28 November 2013 | US 2013/0231460 A1 entire text, all drawings WO 2012/031745 A1 EP 2614148 A CN 103119164 A |
| WO 2005/058453 A1 | 30 June 2005 | US 2007/0111194 A1 EP 1694420 A AT 463294 T |
| US 2019/0105653 A1 | 11 April 2019 | (Family: none) |
| JP 2008-249422 A | 16 October 2008 | (Family: none) |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5728453 B
- WO 2017139153 A
- WO 2013129634 A

- US 6020117 A
- JP 2009150869 A
- JP 2015536375 A